(19) Europäisches Patentamt
European Patent Office
Office européen des brevets

(11) Veröffentlichungsnummer: **0 102 920**
**B1**

# (12) EUROPÄISCHE PATENTSCHRIFT

(45) Veröffentlichungstag der Patentschrift: **21.08.85**

(51) Int. Cl.⁴: **C 07 C 69/732,** C 07 C 69/88, C 07 C 67/03, B 01 J 31/12

(21) Anmeldenummer: **83810311.7**

(22) Anmeldetag: **07.07.83**

## (54) Verfahren zur Herstellung von sterisch gehinderten Hydroxyphenylcarbonsäureestern.

(30) Priorität: **13.07.82 CH 4264/82**

(43) Veröffentlichungstag der Anmeldung: **14.03.84 Patentblatt 84/11**

(45) Bekanntmachung des Hinweises auf die Patenterteilung: **21.08.85 Patentblatt 85/34**

(84) Benannte Vertragsstaaten: **BE DE FR GB IT NL**

(56) Entgegenhaltungen:
**DE - A - 1 543 644**
**DE - A - 2 710 630**

**Patent Abstracts of Japan vol. 1, no. 75 Seite 1475C77**

(73) Patentinhaber: **CIBA-GEIGY AG, Postfach, CH-4002 Basel (CH)**

(72) Erfinder: **Orban, Ivan Dr., Lehenmattstrasse 216, CH-4052 Basel (CH)**
Erfinder: **Troxler, Eduard, Dr., St. Albanring 220, CH-4052 Basel (CH)**

**EP 0 102 920 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft ein Verfahren zur Herstellung von sterisch gehinderten Hydroxyphenylcarbonsäureestern, bei welchem die Umesterung durch sukzessive Behandlung mit einer metallorganischen Verbindung und einer sauren Erde katalysiert wird.

Umesterungsreaktionen zur Herstellung von sterisch gehinderten Hydroxyphenylcarbonsäureestern sind bekannt. So beschreiben z. B. die DE-AS 1 201 349 und die DE-OS 1 543 644 Umesterungsreaktionen dieser Art, bei welchen Alkalialkoholate als Katalysatoren eingesetzt werden. Umesterungsreaktionen der gleichen Art werden nach der DE-OS 2 150 327 mit Lithiumamid katalysiert. Bei all diesen Verfahren entstehen in mehr oder weniger kleinen Mengen Nebenprodukte (meistens Oxydationsprodukte von 2,6-Dialkylphenolen), die selbst in sehr geringen Mengen eine drastische Herabsetzung der Lagerstabilität des gewünschten Endproduktes verursachen. Die unumgängliche Entfernung dieser Nebenprodukte ist zeit-, arbeits- und energieaufwendig. In Patent Abstracts of Japan, Vol. 1, No. 75, S. 1475 C 77 (1977) ist die Verwendung von Diethylzinnoxid als Katalysator von Umesterungsreaktionen von sterisch ungehinderten Hydroxybenzoesäuren beschrieben.

Es wurde nun gefunden, daß die Umesterung bei sukzessiver Behandlung mit katalytischen Mengen einer metallorganischen Verbindung von Metallen der vierten Haupt- oder der vierten Nebengruppe des Periodensystems und einer sauren Erde überraschenderweise zu einer praktisch quantitativen Ausbeute an reinem Endprodukt führt, der, da keine störenden Nebenprodukte enthaltend, nicht zusätzlich gereinigt werden muß.

Die vorliegende Erfindung betrifft demnach ein Verfahren zur Herstellung von Verbindungen der Formel I

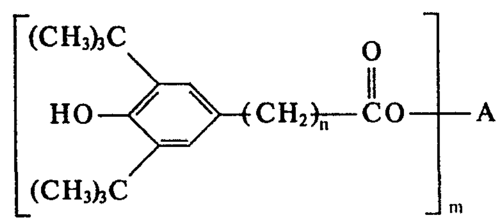

(I)

worin n die Zahlen 0 bis 2, m die Zahlen 1 bis 4 und A einen von einem m-wertigen Alkohol abgeleiteten Rest mit 2 bis 18 Kohlenstoffatomen bedeuten,
durch Umesterung von ca. m Molen eines Esters der Formel II

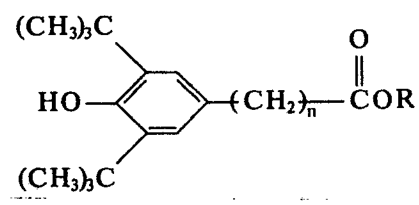

(II)

worin R Methyl oder Äthyl bedeutet, mit einem Alkohol der Formel (III)

$$A-(OH)_m \qquad (III)$$

dadurch gekennzeichnet, daß die Umesterung durch sukzessive Behandlung mit katalytischen Mengen

a) einer metallorganischen Verbindung eines Metalls der vierten Haupt- oder der vierten Nebengruppe des Periodensystems, und
b) einer sauren Erde

katalysiert wird.

A ist als ein von einem m-wertigen aliphatischen Alkohol abgeleiteter Rest ein m-wertiger substituierter oder unsubstituierter aliphatischer Rest mit 2 bis 18 Kohlenstoffatomen.

Ist m 1, so handelt es sich bei A beispielsweise um $C_2-C_{18}$ Alkyl, geradkettig oder verzweigt, wie z. B. Äthyl, n-Propyl, Isopropyl, n-Butyl, sec.-Butyl, tert.-Butyl, n-Pentyl, n-Hexyl, 2-Äthylhexyl, N-Octyl, 1,1,3,3-Tetramethylbutyl, n-Decyl, n-Hexadecyl oder n-Octadecyl. Bevorzugt ist n-Octadecyl.

Ist m 2, so kann A beispielsweise $C_2-C_{18}$ Alkylen, bevorzugt $C_2-C_6$ Alkylen sein, wie Dimethylen, Trimethylen, Tetramethylen, Hexamethylen, 2,2-Dimethyl-trimethylen, Octamethylen, Nonamethylen, Decamethylen, Dodecamethylen oder Octadecamethylen. Die Alkylengruppe kann unterbrochen sein durch —O—, —S— oder —N(R)— wie in 2-Thiapropylen-1,3, 3-Thiapentylen-1,5, 4-Oxaheptamethylen, 3,6-Dioxaoctamethylen-1,8 oder 3,6-Diazaoctamethylen-1,8. Ist A unterbrochenes $C_2-C_6$ Alkylen,

so ist es bevorzugt eine Gruppe

$$-(CH_2)_2-S-(CH_2)_2- \quad \text{oder} \quad -(CH_2)_2-O-(CH_2)_2-O-(CH_2)_2-$$

Ist m 3, so kann A ein trivalenter aliphatischer $C_3H_5$ bis $C_7H_{13}$ Kohlenwasserstoffrest sein, wie

$$-CH_2-\overset{|}{C}H-CH_2- \qquad -CH_2-CH_2-\overset{|}{C}H-CH_2-$$

$$-CH_2-CH_2-\overset{|}{C}H-CH_2-CH_2- \qquad -CH_2-CH_2-\overset{|}{C}H-CH_2-CH_2-CH_2-$$

oder $-CH_2-CH_2-CH_2-\overset{|}{C}H-CH_2-CH_2-CH_2$

Falls m 4 ist, kann ein tetravalenter aliphatischer $C_4H_6$ bis $C_{10}H_{18}$ Kohlenwasserstoffrest sein, wie

$$-CH_2-\overset{|}{C}H-\overset{|}{C}H-CH_2- \qquad -CH_2-CH_2-\overset{|}{C}H-\overset{|}{C}H-CH_2-$$

$$-CH_2-CH_2-\overset{|}{C}H-\overset{|}{C}H-CH_2-CH_2-$$

$$-CH_2-CH_2-\overset{|}{C}H-CH_2-\overset{|}{C}H-CH_2-CH_2-$$

$$-CH_2-CH_2-\overset{|}{C}H-CH_2-CH_2-\overset{|}{C}H-CH_2-CH_2-$$

oder bevorzugt Pentaerythrityl.

Als metallorganische Verbindungen eines Metalls der vierten Haupt- oder der vierten Nebengruppe des Periodensystems kommen beispielsweise in Frage Verbindungen der Formel IV

$$(R_1O)_4-M \qquad (IV)$$

worin $R_1$ $C_1-C_{18}$ Alkyl, Phenyl oder Benzyl bedeutet und M das Element Ge, Zr, Sn oder insbesondere Ti ist oder bevorzugt Verbindungen der Formel V

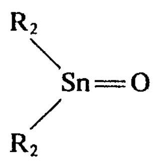

(V)

worin $R_2$ $C_4-C_{18}$ Alkyl bedeutet.

$R_2$ bedeutet als $C_4-C_{18}$ Alkyl z. B. n-Butyl, sec.-Butyl, tert.-Butyl, n-Pentyl, n-Hexyl, n-Octyl, 1,1,3,3-Tetramethylbutyl, n-Decyl, n-Dodecyl, n-Hexadecyl oder n-Octadecyl. $R_1$ bedeutet als $C_1-C_{18}$ Alkyl zusätzlich auch Methyl, Äthyl, n-Propyl oder Isopropyl.
$R_1$ und $R_2$ sind bevorzugt n-Butyl.

Als saure Erden kommen z. B. in Frage: saure Aluminiumsilicate, insbesondere Aluminiumhydrosilicate wie z. B. Zeolithe und Bentonite. Dabei kann Aluminium teilweise durch Eisen und durch Magnesium ersetzt werden. Bevorzugt ist Montmorillonit.

Die Ausgangsstoffe sind bekannt. Sollten einige von ihnen noch neu sein, so können sie in Analogie zu bekannten hergestellt werden.

Das erfindungsgemäße Verfahren ist besonders geeignet für die Herstellung von Verbindungen der Formel I durch Umesterung eines Esters der Formel II mit einem Alkohol der Formel III, worin in den Formeln I, II und III

n die Zahl 2 und m die Zahlen 1, 2 oder 4,
A bei m = 1 $C_2-C_{18}$ Alkyl,
bei m = $C_2-C_6$ Alkylen, eine Gruppe

$$-(CH_2)_2-S-(CH_2)_2- \quad \text{oder} \quad -(CH_2)_2-O-(CH_2)_2-O-(CH_2)_2-$$

und bei m = 4 Pentaerythrityl und
R Methyl bedeuten.

Die metallorganische Verbindung wird zweckmäßig in Mengen von mindestens 0,03, bevorzugt aber zwischen 0,25 und 0,5 Gew.-%, bezogen auf den Ester der Formel II, bei Temperaturen zwischen 130 und 190, bevorzugt 150 und 175°C eingesetzt. Die Behandlung mit der metallorganischen Verbindung dauert 2 bis 5 Stunden.

Die saure Erde wird zweckmäßig in Mengen zwischen 0,3 und 6, bevorzugt zwischen 1 und 5 Gew.-%, bezogen auf den Ester der Formel II, bei Temperaturen zwischen 50 und 150, bevorzugt 90 bis 120°C eingesetzt. Die Behandlung mit der sauren Erde dauert von 15 Minuten bis 3 Stunden.

Bevorzugtes erfindungsgemäßes Verfahren ist die Herstellung von Octadecyl-3-(3,5-di-tert.-butyl-4-hydroxyphenyl)-propionat durch Umesterung von Methyl-3-(3,5-di-tert.-butyl-4-hydroxyphenyl)-propionat mit Octadecanol bei sukzessiver Behandlung mit

a) 0,25 bis 0,5 Gew.-% Dibutylzinnoxid, bezogen auf Methyl-3-(3,5-di-tert.-butyl-4-hydroxyphenyl)-propionat, bei Temperaturen zwischen 150 und 175°C und
b) 1 bis 5 Gew.-% eines Aluminiumhydrosilicates, bezogen auf Methyl-3-(3,5-di-tert.-butyl-4-hydroxyphenyl)-propionat, bei Temperaturen zwischen 90 und 120°C.

Die Umesterung kann unter Vakuum und ohne Lösungsmittel oder bevorzugt bei Atmosphärendruck in Gegenwart eines inerten Lösungsmittels, wie z. B. Toluol oder Xylol durchgeführt werden. Die Menge an Lösungsmittel ist von der gewünschten Reaktionstemperatur abhängig. Bei einer Reaktionstemperatur von 170°C ist z. B. eine Zugabe von 40 bis 50 Gew.-% Toluol, bezogen auf den Ester der Formel II, besonders geeignet. Um höhere Temperaturen zu erreichen, muß die Menge an Lösungsmittel entsprechend reduziert, für niedrigere Temperaturen entsprechend erhöht werden.

Der bei der Umesterung, während der Behandlung mit der metallorganischen Verbindung sich bildende Alkohol wird während der Reaktion laufend abdestilliert.

Die nach der Behandlung mit der sauren Erde erhaltene Suspension wird zur Entfernung der sauren Erde über eine vorgewärmte Hyflo-Nutsche geklärt, wenn nötig abgedampft und die Schmelze erstarren gelassen. Die erstarrte Schmelze braucht nur noch nach üblichen Methoden granuliert zu werden, um das fertige Endprodukt in praktisch reiner, für dessen spezifischen Einsatzbereiter Form zu erhalten.

Die Verbindungen der Formel I sind wertvolle Stabilisatoren für organische Materialien, die der Zersetzung unterwofen sind, zum Beispiel für synthetische organische Polymere, tierische und pflanzliche Öle, Kohlenwasserstoffe, Schmiermittel und dergleichen.

Die folgenden Beispiele erläutern die Erfindung, ohne sie einzuschränken.

## Beispiel 1

In einem 1,5 l Kolben werden unter Stickstoff

292,0 g (1,0 Mol) Methyl-3-(3,5-di-tert.-butyl-4-hydroxyphenyl)-propionat,
275,4 g (1,02 Mol) Stearylalkohol und
130 g Toluol vorgelegt. Das Gemisch wird aufgeheizt und azeotrop entwässert. Man kühlt auf 75°C und gibt
2,6 g Hydrochinon und
1,0 g (0,004 Mol) Dibutylzinnoxid zu. Nun wird auf 170°C geheizt und dabei Methanol über einen mit 75°C warmem Wasser beschickten Dephlegmator abdestilliert. Sobald die Innentemperatur 170°C erreicht hat, wird der Dephlegmator entleert und während weiteren 3 Stunden bei konstanter Innentemperatur von 170°C weiter destilliert. Das dabei abdestillierte Toluol wird mit insgesamt ca. 550 g Neuem ersetzt. Die Lösung wird auf 110°C gekühlt.
10 g Montmorillonit-Erde K-10® werden zugegeben und 30 Minuten bei 107 bis 110°C verrührt, die Suspension über Hyflo geklärt und im Rotationsverdampfer eingedampft. Die zurückgebliebene Schmelze wird in eine Schale gegossen, bei ca. 50°C geimpft und die nach dem Abkühlen erhaltene Kristallmasse zerkleinert.
Man erhält 530 g (99,9% d. Th.) Octadecyl-3-(3,5-di-tert.-butyl-4-hydroxyphenyl)-propionat.
Das Endprodukt enthält ca. 0,1% Methyl-3-(3,5-di-tert.-butyl-4-hydroxyphenyl)-propionat.

## Beispiel 2

In einem 1,5 l Kolben werden unter Stickstoff

292 g (1,0 Mol) Methyl-3-(3,5-di-tert.-butyl-4-hydroxyphenyl)-propionat,
275,4 g (1,02 Mol) Stearylalkohol vorgelegt. Zur Entwässerung wird der Kolben evakuiert und das Reaktionsgemisch 30 Minuten bei 90°C gerührt. Dann wird mit Stickstoff entlastet und

1,0 g (0,004 Mol) Dibutylzinnoxid zugegeben. Der Kolben wird evakuiert und die Schmelze auf 155°C geheizt. Dabei destilliert der größte Teil des Reaktionsmethanols ab. Nun wird stärkeres Vakuum angelegt und 5 Stunden bei 155°C weiter gerührt. Das Gemisch wird auf 110°C gekühlt, mit

10 g Tonsil AC(R) versetzt und während 30 Minuten bei 107 bis 110°C gehalten. Die Suspension wird über eine vorgewärmte Hyflo Nutsche geklärt. Die geklärte Schmelze wird erstarren gelassen und zerkleinert.
Man erhält 525 g (99% d. Th.) Octadecyl-3-(3,5-di-tert.-butyl-4-hydroxyphenyl)-propionat
Das Produkt enthält ca. 1% Methyl-3-(3,5-di-tert.-butyl-4-hydroxyphenyl)-propionat.

**Patentansprüche**

1. Verfahren zur Herstellung von Verbindungen der Formel I

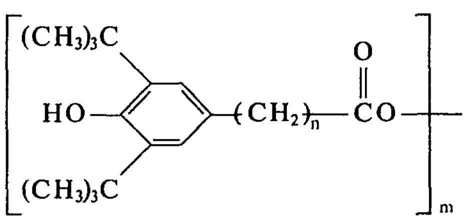

(I)

worin n die Zahlen 0 bis 2, m die Zahlen 1 bis 4 und A einen von einem m-wertigen aliphatischen Alkohol abgeleiteten Rest mit 2 bis 18 Kohlenstoffatomen bedeuten,
durch Umsetzung von ca. m Molen eines Esters der Formel II

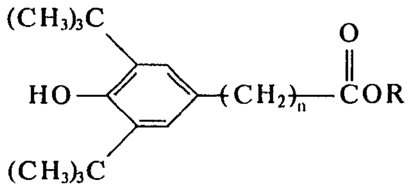

(II)

worin R Methyl oder Äthyl bedeutet, mit einem Alkohol der Formel III

$A-(OH)_m$ (III)

dadurch gekennzeichnet, daß die Umesterung durch sukzessive Behandlung mit katalytischen Mengen

a) einer metallorganischen Verbindung eines Metalls der vierten Haupt- oder der vierten Nebengruppe des Periodensystems, und
b) einer sauren Erde

katalysiert wird.

2. Verfahren gemäß Anspruch 1 zur Herstellung von Verbindungen der Formel I durch Umesterung eines Esters der Formel II mit einem Alkohol der Formel III, wobei in den Formeln I, II und III

n die Zahl 2 und m die Zahlen 1, 2 oder 4,
A bei m = 1 $C_2-C_{18}$ Alkyl,
bei m = 2 $C_2-C_6$ Alkylen, eine Gruppe

$-(CH_2)_2-S-(CH_2)_2-$ oder $-(CH_2)_2-O-(CH_2)_2-O-(CH_2)_2-$

und bei m = 4 Pentaerythrityl und
R Methyl bedeuten.

3. Verfahren gemäß Anspruch 1, dadurch gekennzeichnet, daß als metallorganische Verbindung eine Zinnverbindung der Formel V

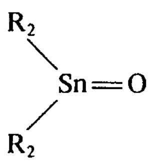

(V)

worin $R_2$ $C_4-C_{18}$ Alkyl bedeutet, eingesetzt wird.

4. Verfahren gemäß Anspruch 1, dadurch gekennzeichnet, daß als saure Erde ein saures Aluminiumsilicat eingesetzt wird.

5. Verfahren gemäß Anspruch 1, dadurch gekennzeichnet, daß die metallorganische Verbindung in einer Menge von mindestens 0,03 Gew.-%, bezogen auf den Ester der Formel II eingesetzt wird.

6. Verfahren gemäß Anspruch 1, dadurch gekennzeichnet, daß die saure Erde in einer Menge zwischen 0,3 und 6 Gew.-%, bezogen auf den Ester der Formel II eingesetzt wird.

7. Verfahren gemäß Anspruch 1, dadurch gekennzeichnet, daß die Behandlung mit der metallorganischen Verbindung bei Temperaturen zwischen 130 und 190° C stattfindet.

8. Verfahren gemäß Anspruch 1, dadurch gekennzeichnet, daß die Behandlung mit der sauren Erde bei Temperaturen zwischen 50 und 150° C stattfindet.

9. Verfahren gemäß Anspruch 1 zur Herstellung von Octadecyl-3-(3,5-di-tert.-butyl-4-hydroxyphenyl)-propionat durch Umesterung von Methyl-3-(3,5-di-tert.-butyl-4-hydroxyphenyl)-propionat mit Octadecanol, daduch gekennzeichnet, daß die Umesterung durch Behandlung mit

a) 0,25 bis 0,5 Gew.-% Dibutylzinnoxid, bezogen auf Methyl-3-(3,5-di-tert.-butyl-4-hydroxyphenyl)-propionat, bei Temperaturen zwischen 150 und 175° C und
b) 1 bis 5 Gew.-% eines Aluminiumhydrosilicates, bezogen auf Methyl-3-(3,5-di-tert.-butyl-4-hydroxyphenyl)-propionat, bei Temperaturen zwischen 90 und 120° C

katalysiert wird.

## Claims

1. A process for the preparation of a compound of the formula I

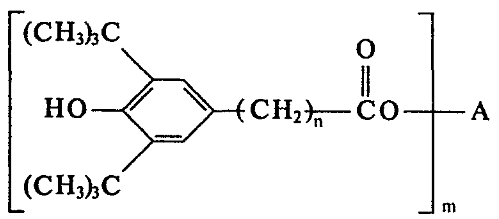

(I)

in which n is a value from 0 to 2, m is a value from 1 to 4 and A is a radical which has 2 to 18 carbon atoms and is derived from an m-hydric aliphatic alcohol, by transesterifying about m moles of an ester of the formula (II)

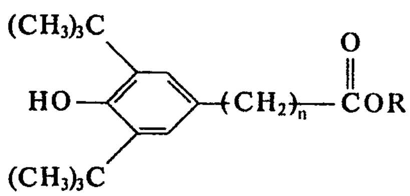

(II)

in which R is methyl or ethyl, with an alcohol of the formula (III)

$$A-(OH)_m \qquad (III)$$

which process comprises catalysing the transesterification by successive treatment with catalytic amounts of a) an organometallic compound of a metal of the fourth main group or the fourth sub-group of the periodic system and b) an acid earth.

2. A process according to claim 1 for the preparation of a compound of the formula I by transesterifying an ester of the formula II with an alcohol of the formula III, in which formulae I, II and III

n is the value 2 and m is the value 1, 2 or 4,
A is $C_2-C_{18}$ alkyl, where m is 1, or
is $C_2-C_6$ alkylene or a

$-(CH_2)_2-S-(CH_2)_2-$ or $-(CH_2)_2-O-(CH_2)_2-O-(CH_2)_2-$ group,

where m is 2, or
is pentaerythrityl, where m is 4, and
R is methyl.

3. A process according to claim 1, wherein the organometallic compound is a tin compound of the formula V

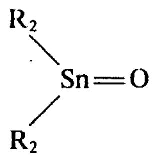

(V)

in which $R_2$ is $C_4-C_{18}$ alkyl.

4. A process according to claim 1, wherein the acid earth is an acid aluminium silicate.

5. A process according to claim 1, wherein the organometallic compound is employed in an amount of not less than 0.03% by weight, based on the ester of the formula II.

6. A process according to claim 1, wherein the acid earth is employed in an amount from 0.3 to 6% by weight, based on the ester of the formula II.

7. A process according to claim 1, wherein the treatment with the organometallic compound is carried out at a temperature in the range from 130 to 190° C.

8. A process according to claim 1, wherein the treatment with the acid earth is carried out at a temperature in the range from 50 to 150° C.

9. A process according to claim 1 for the preparation of octadecyl 3-(3,5-di-tert.-butyl-4-hydroxyphenyl)propionate by transesterifying methyl 3-(3,5-di-tert.-butyl-4-hydroxyphenyl)propionate with octadecanol, which process comprises catalysing the transesterification by treatment with a) 0.25 to 0.5% by weight, based on the methyl 3-(3,5-di-tert.-butyl-4-hydroxyphenyl)propionate, of dibutyltin oxide at a temperature in the range from 150 to 175°C and b) 1 to 5% by weight, based on the methyl 3-(3,5-di-tert.-butyl-4-hydroxyphenyl)propionate, of an aluminium hydrosilicate at a temperature in the range from 90 to 120° C.

## Revendications

1. Procédé pour préparer des composés répondant à la formule I:

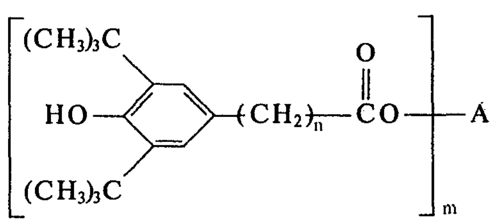

(I)

dans laquelle n représente un nombre de 0 à 2, m représente un nombre de 1 à 4 et A représente un radical contenant de 2 à 18 atomes de carbone et provenant d'un alcool aliphatique de fonctionnalité égale à m, par transestérification d'environ m mol d'un ester répondant à la formule II:

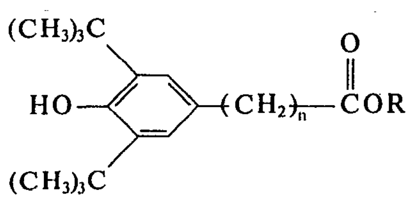

(II)

dans laquelle R représente un radical méthyle ou éthyle, avec un alcool de formule III:

$$A-(OH)_m \qquad (III)$$

procédé caractérisé en ce qu'on catalyse la transestérification en traitant successivement par des quantités catalytiques:

a) d'un composé organo-métallique d'un métal du 4ème groupe principal ou du 4ème sous-groupe de la classification périodique, et
b) d'une terre acide.

2. Procédé selon la revendication 1 pour la préparation de composés de formule I par transestérification d'un ester de formule II avec un alcool de formule III, procédé selon lequel les symboles présents dans les formules I, II et III ont les significations suivantes:

n représente le nombre 2,
m représente un nombre égal à 1, à 2 ou à 4,
A représente, dans le cas où m est égal à 1, un alkyle en $C_2-C_{18}$, dans le cas où m est égal à 2 un alkylène en $C_2-C_6$, un radical

$$-(CH_2)_2-S-(CH_2)_2-$$

ou un radical

$$-(CH_2)_2-O-(CH_2)_2-O-(CH_2)_2-$$

et, lorsque m est égal à 4, un radical pentaérythrityle, et
R représente un radical méthyle.

3. Procédé selon la revendication 1 caractérisé en ce qu'on utilise, comme composé organo-métallique, un composé de l'étain répondant à la formule V:

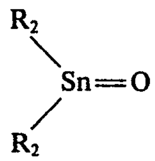

(V)

dans laquelle $R_2$ représente un alkyle en $C_4-C_{18}$.

4. Procédé selon la revendication 1 caractérisé en ce qu'on utilise, comme terre acide, un silicate d'aluminium acide.

5. Procédé selon la revendication 1 caractérisé en ce que le composé organo-métallique est mis en jeu en une quantité d'au moins 0,03% en poids par rapport à l'ester de formule II.

6. Procédé selon la revendication 1 caractérisé en ce que la terre acide est mise en jeu en une quantité comprise entre 0,3 et 6% en poids par rapport à l'ester de formule II.

7. Procédé selon la revendication 1 caractérisé en ce que le traitement par le composé organo-métallique est effectué à des températures comprises entre 130 et 190° C.

8. Procédé selon la revendication 1 caractérisé en ce que le traitement par la terre acide est effectué à des températures comprises entre 50 et 150° C.

9. Procédé selon la revendication 1 pour la préparation du (ditert-butyl-(3,5 hydroxy-4 phényl)-3 propionate d'octadécyle par transestérification du (ditert-butyl-3,5-hydroxy-4 phényl)-3 propionate de méthyle avec l'octadécanol, procédé caractérisé en ce qu'on catalyse la transestérification en traitant par:

a) de 0,25 à 0,5% en poids d'oxyde de dibutylétain, par rapport au (ditert-butyl-3,5 hydroxy-4 phényl)-3 propionate de méthyle, à des températures comprises entre 150 et 175° C, et
b) de 1 à 5% en poids d'un hydrosilicate d'aluminium, par rapport au (ditert-butyl-3,5 hydroxy-4 phényl)-3 propionate de méthyle, à des températures comprises entre 90 et 120° C.